# EUROPEAN PATENT APPLICATION

(11) **EP 1 568 707 A1**
(43) Date of publication of application: **31.08.2005**
(21) Application number: 04075587.8
(22) Date of filing: 26.02.2004
(51) Int. Cl.: C07K 7/06, C07K 5/10, C07K 5/08, A23J 3/34, A61K 38/06, A61P 9/12, C12P 21/06

(54) **Antihypertensive peptides from casein hydrolysates**

(71) Applicant: PULEVA BIOTECH, S.A., E-18004 Granada (ES)
(72) Inventor: Geerlings, Arjian, 18004 Granada (ES); Hidalgo Zarco, Fernando, 18004 Granada (ES); Boza Puerta, Julio, 18004 Granada (ES); Jiménez Lopez, Jesus, 18004 Granada (ES)
(74) Representative: Portela Gasch, Sergio Raul

(57) **Abstract**

The present invention refers to novel antihypertensive peptides which may be used as active ingredients in pharmaceutical preparations, dietary supplements, or as food ingredients. The peptides have/comprise the sequence YGPIPN, or SLPE, or SEPK, or fragments thereof and one of between 3 and 8 residues.

SEQUENCE LISTING

<110> Puleva Biotech, S.A.

<120> Antihypertensive peptides

<160> 9

<170> PatentIn version 3.1

<210> 1
<211> 6
<212> PRT
<213> Artificial sequence

<400> 1
Tyr Gly Pro Ile Pro Asn
1               5

<210> 2
<211> 4
<212> PRT
<213> Artificial sequence

<400> 2
Ser Leu Pro Glu
1

<210> 3
<211> 4
<212> PRT
<213> Artificial sequence

<400> 3
Ser Glu Pro Lys
1

<210> 4
<211> 8
<212> PRT
<213> Artificial sequence

<400> 4
Phe Tyr Gly Pro Ile Pro Asn Ser
1               5

<210> 5
<211> 6
<212> PRT
<213> Artificial sequence

<400> 5
Asn Ser Leu Pro Glu Asn
1               5

<210>  6
<211>  6
<212>  PRT
<213>  Artificial sequence

<400>  6
Leu Ser Glu Pro Lys Val
 1               5

<210>  7
<211>  5
<212>  PRT
<213>  Artificial sequence

<400>  7
Gly Pro Ile Pro Asn
1               5

<210>  8
<211>  3
<212>  PRT
<213>  Artificial sequence

<400>  8
Leu Pro Glu
1

<210>  9
<211>  3
<212>  PRT
<213>  Artificial sequence

<400>  9
Glu Pro Lys
1

## Description

### FIELD OF THE INVENTION

This invention relates to novel antihypertensive peptides that may be used as active ingredients in pharmaceutical preparations, dietary supplements, or as food ingredients.

### BACKGROUND OF THE INVENTION

Hypertension, or high blood pressure, is a disease which affects approximately 170 million people worldwide. It is clinically defined as a systolic arterial blood pressure of 140 mm Hg or higher and a diastolic arterial blood pressure of 90 mm Hg or higher. Hypertension is a serious threat for the population since in many cases it is the cause of coronary disease, stroke and myocardial infarction.

Although the cause of hypertension in most cases is unknown, one regulator of hypertension, the renin-angiotensin mechanism, is well studied. In this mechanism, a peptide called angiotensin I is cleaved by the action of angiotensin converting enzyme (ACE). The reaction product, angiotensin II, is a strong vasoconstrictor. Angiotensin II induces hypertension by stimulating the contraction of the smooth muscles in blood vessel walls. Moreover, angiotensin II stimulates the breakdown of bradykinin, a peptide that lowers blood pressure. Therefore, blocking the enzyme ACE not only prevents the formation of the hypertension-provoking peptide angiotensin II, but it also inhibits the decomposition of blood pressure lowering peptides (bradykinin). Inhibiting this enzyme prevents these processes and therefore inhibitors of ACE have anti-hypertensive properties.

Several pharmaceutical compounds are on the market to treat hypertension. Examples of these so-called ACE-inhibitors are; benazepril (Lotensin); captopril (Capoten); captopril/hydrochlorothizaide (Capozide); enalapril maleate (Vasotec); fosinopril sodium (Monopril); lisinopril (Prinivil, Zestril); quinapril/magnesium carbonate (Accupril); ramipril (Altace); trandolapril (Mavik). Although these pharmaceutical preparations are effective in lowering blood pressure, the downside to these ACE inhibitors are their side effects including the development of a dry nighttime cough, dizziness, light-headedness, and headache. ACE inhibitors can also cause potassium build-up and kidney problems, so potassium levels and kidney function should be monitored. Additionally, all of the ACE inhibitors appear to be capable of producing a severe allergic reaction that can be life-threatening.

Alternatively, several peptides produced from food stuffs have been studied for their potential to treat hypertension. These peptides inhibit ACE and do not appear to produce any side effects according to human safety studies. A daily dosage of up to 30 grams per day did not induce any side effect including the dry night time cough so typical with the ACE inhibitor drugs.

Several foods have been used for the isolation of peptides that inhibit ACE. For instance, peptides have been identified from the protein fraction of bovine milk, soy, chicken muscle, and tuna fish. A few protein hydrolysates containing ACE-inhibiting peptides are already on the market and sold as dietary supplements or food ingredients.

Peptides derived from milk proteins are probably the best studied for their ACE-inhibiting properties. Several peptides have been isolated and studied in hypertensive rats and even some in humans (for a review see FitzGerald and Meisel 2000, British Journal of Nutrition 84, S33-S37). Examples of milk-derived peptides with anti-hypertensive activity are Val-Pro-Pro (VPP) and Ile- Pro-Pro (IPP) produced during milk fermentation. These ACE-inhibiting peptides reduce hypertension in hypertensive rats and hypertensive humans.

However, most of the ACE-inhibiting peptides found until now do not reduce hypertension because they are not absorbed or broken down in the digestive tract. Therefore, being an inhibitor of ACE is not enough for a peptide to be able to reduce hypertension in a hypertensive subject; it should also be bioavailable in order to reach intact the renin-angiotensin mechanism of the smooth muscles of blood vessel walls. Thus, it would be desirable to identify novel ACE-inhibiting peptides that are absorbed intact and exercise their anti-hypertensive activity in a hypertensive subject.

### SUMMARY OF THE INVENTION

Accordingly, the present invention provides in one of its aspects novel food-derived peptides with anti-hypertensive activity. The ACE-inhibiting peptides included in this invention are SEQ ID NO: 1, SEQ ID NO: 2 and SEQ ID NO: 3, peptides comprising said amino acid sequences having no more than 8 amino acids, fragments of these peptides as well as addition salts, esters, solvates and anhydrates of said peptides and fragments thereof. Peptides SEQ ID NO: 1, SEQ ID NO: 2 and SEQ ID NO: 3 have been isolated and characterized from a hydrolyzed casein fraction of both goat and sheep milk.

Another aspect of this invention is to provide anti-hypertensive compositions comprising one or more of the peptides selected from the group comprising SEQ ID NO: 1, SEQ ID NO: 2, SEQ ID NO: 3, peptides comprising these amino acid sequences, and fragments of these peptides, as well as addition salts, esters, solvates and anhydrates of said peptides and fragments thereof.

It is yet another aspect of this invention to provide pharmaceutical compositions comprising one or more of the peptides selected from the group comprising SEQ ID NO: 1, SEQ ID NO: 2, SEQ ID NO: 3, peptides comprising these amino acid sequences, and fragments of these peptides, or their acceptable addition salts, esters, solvates and anhydrates .

Yet a further aspect of this invention is to provide food compositions and protein hydrolysates containing anti-hypertensive compositions comprising one or more of the peptides selected from the group comprising SEQ ID NO: 1, SEQ ID NO: 2, SEQ ID NO: 3, peptides comprising these amino acid sequences, and fragments of these peptides, or their acceptable addition salts, esters, solvates and anhydrates.

Another aspect of this invention is to provide methods of reducing or preventing cardiovascular diseases associated with hypertension like stroke, coronary heart disease, myocardial infarction, metabolic syndrome, peripheral vascular disease or abdominal aortic aneurysm which comprises administering an effective amount of peptides selected from the group comprising SEQ ID NO: 1, SEQ ID NO: 2, SEQ ID NO: 3, peptides comprising these amino acid sequences, and fragments of these peptides, or their acceptable addition salts, esters, solvates and anhydrates.

A final aspect of this invention is a process for the preparation of the peptides and hydrolysates of the present invention.

### DESCRIPTION OF THE DRAWINGS

FIGURE 1: HPLC chromatogram obtained during purification of the peptides included in this invention (SEQ ID NO: 1, SEQ ID NO: 2 and SEQ ID NO: 3). Arrowheads indicate fractions, containing ACE inhibiting activity, used for microsequencing.
FIGURE 2: Effect of the composition of example 1 on systolic blood pressure in spontaneous hypertensive rats. Open circles indicate the systolic blood pressure in the control group, and the closed squares represent the group consuming the composition of example 1. Data are mean ± SEM (n = 10 in each group).

### DETAILED DESCRIPTION OF THE INVENTION

The peptides included in the first aspect of this invention are:
Peptides comprising an amino acid sequence selected from the sequences shown in SEQ ID NO: 1, SEQ ID NO: 2 and SEQ ID NO: 3, having no more than 8 amino acids.
Peptides having an amino acid sequence selected from the sequences shown in SEQ ID NO: 4, SEQ ID NO: 5 and SEQ ID NO: 6.
Peptides having an amino acid sequence selected from the sequences shown in SEQ ID NO: 1, SEQ ID NO: 2 and SEQ ID NO: 3.
Fragments of peptides having an amino acid sequence selected from the sequences shown in SEQ ID NO: 1, SEQ ID NO: 2 and SEQ ID NO: 3.
Fragment of peptides having an amino acid sequence selected from the sequences shown in SEQ ID NO: 7, SEQ ID NO: 8 and SEQ ID NO: 9.

Also within the first aspect of the invention are included derivatives of the peptides, such as acid or base addition salts, in particular pharmaceutically acceptable salts, esters, solvates and anhydrates of the peptides previously mentioned.

In the peptides and fragments thereof referred above, Tyr stands for the amino acid L-tyrosine, Gly for L-glycine, Pro for L-proline, Ile for L-isoleucine, Asn for L-asparagine, Ser for L-serine, Leu for L-leucine, Val for L-valine, Phe for L-phenylalanine, Glu for L-glutamate, and Lys for L-lysine. All the peptides included in this invention are novel peptides, which inhibit the enzyme angiotensin converting enzyme (ACE), and are absorbed intact from the digestive tract, thereby preventing or reducing hypertension. For this purpose, one of these peptides can be used at the therapeutic amount or any combination of these peptides can be used. The effective amount has been determined for these peptides and was found to be between 2 mg and 200 mg per kg body weight per day, and preferably between 10 mg and 50 mg per kg body weight per day. The effective amount referred to is the sum of the peptides included in this invention, and this effective amount may reduce systolic blood pressure by 10 mm Hg or more in an hypertensive subject.

A second aspect of the invention includes compositions comprising at least one peptide of the invention or derivative thereof. In particular, said compositions are pharmaceutical compositions comprising at least a peptide of the invention or a pharmaceutically acceptable derivative thereof and a pharmaceutically acceptable carrier, binder and/or auxiliary material. Said peptides or derivatives thereof are preferably present in the compositions in an effective amount to reduce hypertension in mammals. The carrier materials, binders and/or auxiliary materials must be pharmaceutically and pharmacologically tolerable, so that they can be combined with the other components of the formulation or preparation and do not exert adverse effects on the organism treated. The pharmaceutical compositions can be in the form of single doses. The compositions are prepared according to methods known in the field of pharmacology. The appropriate quantities of active substances suitable for administration may vary as a function of the particularly field therapy. In general, the active substance concentration in a single-dose formulation is 5% to 95% of the total formulation. The peptides or derivatives thereof of the invention are preferably present in the compositions in an effective amount to reduce hypertension in mammals.

A further aspect of the invention consists in food compositions comprising at least one peptide or derivative thereof. Said peptides or derivatives thereof are preferably present in the compositions in an effective amount to reduce hypertension in mammals. Preferred food compositions are selected from: a beverage, infused food, milk, yogurt, cheese, flavored milk drink, bread, cake, butter, margarine, a sauce, a condiment, a salad dressing, fruit juice, syrup, a dessert, icings and fillings, a soft frozen product, a confection, a chewing gum and an intermediate food. Also within the food compositions, the present invention contemplates a protein hydrolysate, obtained from goat or sheep milk, enriched in at least one of the peptides or derivatives thereof of the invention. In a preferred protein hydrolysate according to the invention, the combined amount of the peptides or salts thereof of claims 1 to 6 is between 0,1% and 5%, preferably between 1% and 2% (in dry weight) of the protein hydrolysate. It is clear that the use of the peptides and derivatives thereof, as well as the protein hydrolysates of the invention can be employed in the preparation of food compositions, which include dietary supplements and food ingredients.

The peptides included in this invention can be administered in a substantial pure form to a person in need thereof or can be given as part of a more complex composition. A substantial pure form of the sum of the peptides is defined as a purity of at least 50% in weight. When the peptides are given to a person in need thereof as part of a more complex mixture, the amount of this mixture given to this person should be such that the sum of peptides mentioned in this invention reaches the effective amount. Examples of mixtures that contain these peptides or to which the peptides might be added are dried protein extracts, hydrolyzed protein extracts, dried plant extracts, cacao powder, a dried food composition, etc. A person in need of mentioned peptides, can consume the peptides of this invention in a pure form as a pharmaceutically composition, as part of a composition containing these peptides, for instance as a dietary supplement, or as part of a food matrix whereto these peptides have been added in an effective amount. Examples of food matrixes to which an anti-hypertensive composition containing the peptides of this invention can be added are: beverages, infused foods, milk, yogurts, cheese, flavored milk drinks, bread, cake, butter, margarine, sauces, condiments, salad dressings, fruit juices, syrups, desserts, icings and fillings, soft frozen products, confections, chewing gum and intermediate food.

A further aspect of the invention consists in the peptides or derivatives thereof of the invention, compositions or hydrolysates of the invention for use as a medicament; in particular, for use in the treatment or prophylaxis of hypertension, stroke, coronary disease, myocardial infarction, metabolic syndrome, peripheral vascular disease or abdominal aortic aneurysm in mammals. Similarly, the peptides or derivatives thereof of the invention can be employed in the manufacture of a medicament, in particular in the manufacture of a medicament for the treatment or prophylaxis of hypertension, stroke, coronary disease, myocardial infarction, metabolic syndrome, peripheral vascular disease or abdominal aortic aneurysm in mammals.

Another aspect of this invention is a process for the preparation of the peptides of the present invention. Thus, the peptides can be prepared by a process which comprises the steps of:
a) Obtaining the casein fraction of goat or sheep milk, preferably skimmed milk. Said casein fraction is preferably obtained by microfiltration or by acidification.
b) Hydrolyzing the casein fraction with a protease. Depending on the peptides to be prepared, different proteases should be employed. Thus, in order to obtain peptides having the sequences SEQ ID NO: 1, SEQ ID NO: 2 or SEQ ID NO: 3, subtilisin or an equivalent protease is employed; subtilisin enzyme from any *Bacillus* species is useful, for instance from *Bacillus amyloloquefaciens.* On the other hand, in order to obtain peptides comprising the sequences SEQ ID NO: 1, SEQ ID NO: 2 or SEQ ID NO: 3, in particular peptides having the sequences SEQ ID NO: 4, SEQ ID NO: 5 or SEQ ID NO: 6 or fragments of said peptides, in particular fragments having the sequence SEQ ID NO: 7, SEQ ID NO: 8 or SEQ ID NO: 9, proteases such as trypsin, chymotrypsin or thermolysin are employed.
c) After the hydrolysis step, the protease is inactivated, preferably by a heat treatment, for instance by heating the hydrolysate to 90°C for 10 minutes, or by acidification.
d) The peptides of the invention can then be isolated and purified by methods such as ultrafiltration, nanofiltration, chromatography or electrodialysis.

The peptides of the invention can also be prepared by solid-phase synthesis methods (Atherton and Sheppard (1989). Solid Phase Peptide Synthesis: A Practical Approach, Oxford, IRL Press).

Yet another aspect of the invention consists in the preparation of the protein hydrolysate of the invention. Thus, said hydrolysate can be prepared by a process which comprises the steps of:
a) Obtaining the casein fraction of goat or sheep milk, preferably skimmed milk. Said casein fraction is preferably obtained by microfiltration or by acidification.
b) Hydrolyzing the casein fraction with a protease. Depending on the composition of the hydrolysate, different proteases should be employed.
c) After the hydrolysis step, the protease is inactivated, preferably by a heat treatment, for instance by heating the hydrolysate to 90°C for 10 minutes, or by acidification.
d) The mixture is then enriched in the peptides of the invention.
e) Optionally, the hydrolysate is dried to obtain a powder.

The following examples illustrate the invention:

### EXAMPLE 1

### PREPARATION OF AN ANTI-HYPERTENSIVE COMPOSITION

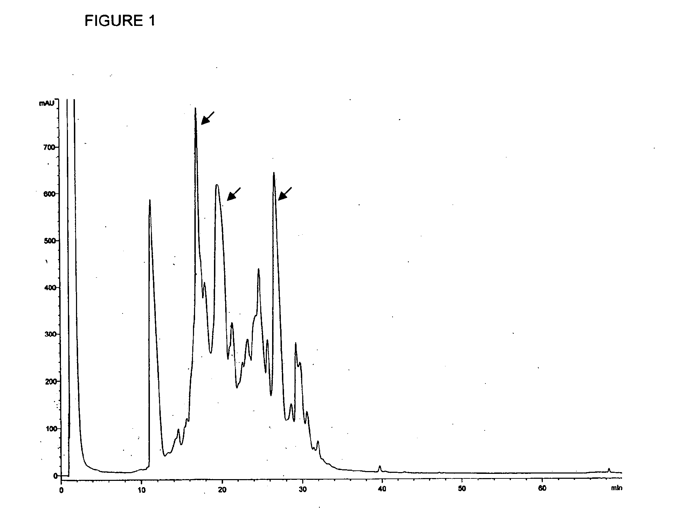

The casein fraction of 500 L of goat milk was obtained by adjusting the milk to pH 3.0 and whey proteins were removed by centrifugation. The casein proteins were concentrated by evaporation and brought to 10 g of protein per L. Then, the protease subtilisin from *Bacillus amyloliquefaciens* (Multifect P-3000 from Genencor, 1 % in volume) was added to the casein solution and incubated at 55°C for 3 hours. The reaction was stopped by heating (10 minute at 100°C) and the mixture was finally dried. Peptides were purified by HPLC and identified by microsequencing as described (Matsudaira P. A Practical Guide to Protein and Peptide Purification for Microsequencing. Academic Press 1993). Briefly, the casein hydrolysate was fractionated by column chromatography on an AKTA Explorer (Amersham Biosciences) using a Resource RP (Amersham Biosciences) column and a linear gradient of water-acetonitril (0-100%, 0,2 ml flow rate). Fractions of 1 ml were collected and ACE inhibition measurements were performed by a standard enzyme assay (Wu et al. 2002 J. Chromatography 950, 125-130). Fraction number 17 showed high ACE inhibiting activity, and was used in a second purification procedure. This time, separation of peptides was accomplished on HPLC (Waters) using the following conditions; Zorbax 17 column material, trifluoracetic acid as eluent, and a flow rate of 0,5 ml/min. A typical separation is demonstrated in Figure 1. Fractions of 0,5 ml were collected and their ACE inhibiting capacity determined. Three fractions (see Figure 1) were found to have ACE inhibiting activity and were used for microsequencing (performed by Eurosequence, the Netherlands). In this manner, the following novel ACE-inhibiting peptides were identified in the casein hydrolysate: SEQ ID NO: 1, SEQ ID NO: 2 and SEQ ID NO: 3. All three are peptides derived from ß-casein, SEQ ID NO: 1 (ß-CN f78-83), SEQ ID NO: 2 (ß-CN f84-87), and SEQ ID NO: 3 (ß-CN f181-184). The amount of each peptide in the obtained hydrolysate was determined by HPLC equipped with a mass detector (Waters). A standard curve for each peptide was determined using pure synthesized peptide. The amount of each peptide within the hydrolysate was approximately: SEQ ID NO: 1; 0.30% in weight, SEQ ID NO: 2; 0.28% in weight, and SEQ ID NO: 3; 0.19% in weight. The procedure described in this example was repeated with 500 L of sheep milk and 500 L of bovine milk. All three peptides were identified in the hydrolysate obtained from sheep milk (SEQ ID NO: 1 (ß-CN f78-83); 0.39% in weight, SEQ ID NO: 2 (ß-CN f84-87); 0.36% in weight, and SEQ ID NO: 3 (ß-CN f181-184); 0.25% in weight), but were absent in the hydrolysate obtained from bovine milk. The hydrolysate obtained from goat milk was used in the experiments described in examples 4, 5, and 6.

### EXAMPLE 2

### INHIBITION OF ANGIOTENSIN CONVERTING ENZYME

In order to determine IC50 values for the peptides included in this invention, pure peptides (purity of > 95% in weight) were prepared by custom solid-phase synthesis on a 10 µmol scale and subsequently purified by HPLC (synthesis and purification performed by Eurosequence, the Netherlands). Purified ACE enzyme (Sigma) was used for the ACE assay (Wu et al. 2002 J. Chromatography 950, 125-130). The peptides having the amino acid sequences shown in SEQ ID NO: 1, SEQ ID NO: 2 and SEQ ID NO: 3 were found to be strong inhibitors of ACE (see table below). The peptides comprising the amino acid sequences shown in SEQ ID NO: 1, SEQ ID NO: 2 and SEQ ID NO: 3, especially the peptides having the amino acid sequences shown in SEQ ID NO: 4, SEQ ID NO: 5 and SEQ ID NO: 6, are also good ACE inhibitors (see table below). Finally, the peptides which are fragments of the amino acid sequences shown in SEQ ID NO: 1, SEQ ID NO: 2 and SEQ ID NO: 3, respectively SEQ ID NO: 7, SEQ ID NO: 8 and SEQ ID NO: 9 were tested for their ACE inhibiting activity. As can be judged from their IC50 values, these peptides are also good ACE inhibitors (see table below).

| **Peptide** | **IC50 (µM)** |
|---|---|
| SEQ ID NO: 1 | 317 |
| SEQ ID NO: 2 | 329 |
| SEQ ID NO: 3 | 354 |
| SEQ ID NO: 4 | 435 |
| SEQ ID NO: 5 | 369 |
| SEQ ID NO: 6 | 397 |
| SEQ ID NO: 7 | 287 |
| SEQ ID NO: 8 | 337 |
| SEQ ID NO: 9 | 364 |

### EXAMPLE 3

### BIOAVAILABILITY OF ANTIHYPERTENSIVE PEPTIDES

Peptides SEQ ID NO: 1, SEQ ID NO: 2 and SEQ ID NO: 3 (purity of > 95% in weight, 500 µg each) were orally administered to mice (Balb/C, 3 months of age). After 2 hours, mice were sacrificed and a blood sample collected. After centrifugation of the blood sample (5 min, 3000g) plasma was collected, dried and resuspended in ethyl acetate. The samples were then analyzed by HPLC-MS, and the amount of the peptides of this invention present in the plasma samples calculated using standard curves. Two hours after their oral administration, all three peptides were found in the plasma samples in the following amounts: SEQ ID NO: 1: 7,5 µg/ml, SEQ ID NO: 2: 1,2 µg/ml, and SEQ ID NO: 3: 0,9 µg/ml. These results demonstrate that the peptides of this invention are bioavailable and are absorbed intact from the digestive tract.

### EXAMPLE 4

### BLOOD-PRESSURE LOWERING EFFECT IN HYPERTENSIVE RATS

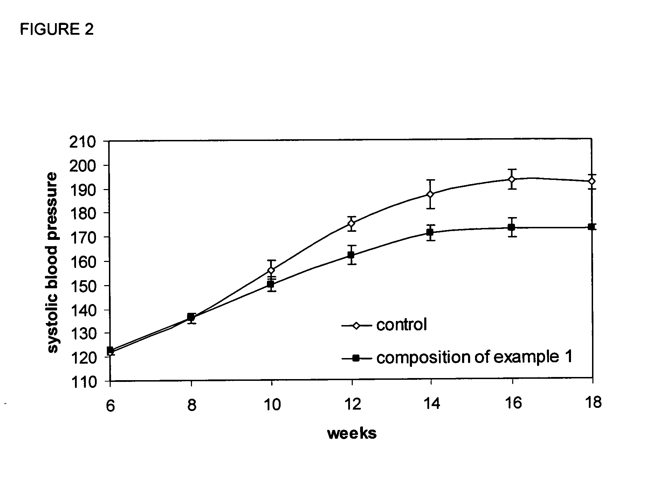

Spontaneous hypertensive rats (females) were used in this experiment. At 6 weeks of age ten rats received the composition of example 1 (goat casein hydrolysate rich in peptides having the sequences SEQ ID NO: 1, SEQ ID NO: 2 and SEQ ID NO: 3) dissolved in their drinking water during the time of the study. It was estimated that these rats ingested approximately between 70 and 90 mg of the composition of example 1 per day. Another 10 rats were used as controls. Both groups of rats had access to their feed (normal chow) and drinking water *ad libitum.* Systolic blood pressure was measured (standard tail cuff method) at the beginning of the study and after every 2 weeks. Figure 2 shows the progression of systolic blood pressure in both groups during the time of the study. In control rats, from age 6 weeks on, systolic blood pressure steadily increased until week 14 when it reached a steady state. In the group of rats that consumed the composition as described as in example 1, systolic blood pressure increased as well until week 14, but the increase was significantly less (p<0.005 for week 14). Also from week 14 on, systolic blood pressure was significantly less than the control group (p<0.001). These results clearly show that the composition of example 1 containing the peptides of this invention, provokes a reduction in systolic blood pressure in spontaneous hypertensive rats. We conclude that the composition of example 1 is an anti-hypertensive composition.

### EXAMPLE 5

### BLOOD-PRESSURE LOWERING EFFECT IN HYPERTENSIVE PERSONS

Forty un-medicated, non-smoking, hypertensive men (20) and women (20) were randomized in two groups. Twenty individuals were instructed to take 25 grams of the composition of example 1 (goat casein hydrolysate rich in peptides having the sequences SEQ ID NO: 1, SEQ ID NO: 2 and SEQ ID NO: 3) daily with water for five weeks and to maintain their normal lifestyle habits. The remaining 20 volunteers were instructed to take 25 grams of a placebo (milk powder) daily with water for five weeks and to maintain their normal lifestyle habits. Participants reported to the General Clinical Research Clinic weekly in the early morning for blood pressure checks, blood draws, side effect assessment, diet assessment, and general monitoring.

A significant drop in both systolic and diastolic blood pressure occurred after 10 days of treatment and persisted throughout the study. In contrast to the control group taking placebo, the volunteers taking the composition of example 1 had, on average, a reduced systolic blood pressure (average of 12 mmHg reduction) and diastolic blood pressure (average of 8 mmHg reduction). Treatment related side effects including hepatic and renal function did not differ between both groups.
Based on these findings, the composition of example 1 containing the anti-hypertensive peptides described in this invention reduces both systolic and diastolic blood pressure in untreated hypertensives.

### EXAMPLE 6

### PREPARATION OF A FOOD STUFF CONTAINING AN ANTI-HYPERTENSIVE COMPOSITION

An enriched juice was prepared using the following ingredients:

| **Ingredient** | **Amount per kg** |
|---|---|
| Concentrated juice | 200 g |
| Anti-hypertensive composition of example 1 | 20 g |
| Insoluble fiber | 10 g |
| Lecithin | 0.5 g |
| Flavors | 2 g |
| Vitamin C | 90 mg |
| Vitamin B1 | 2.1 mg |
| Vitamin B2 | 2.4 mg |
| Vitamin B6 | 3 mg |
| Vitamin B12 | 1.5 µg |
| Vitamin A | 1.2 mg |
| Vitamin D | 7.5 µg |
| Folic acid | 300 µg |

### Processing technology

The final product was prepared from a concentrated juice by addition of water and water soluble ingredients. Then, the anti-hypertensive composition of example 1 (goat casein hydrolysate rich in peptides having the sequences SEQ ID NO: 1, SEQ ID NO: 2 and SEQ ID NO: 3) was added and mixed and the resulting product was pasteurized and homogenized. Finally, the product was cooled and packaged.

## Claims

1. A peptide comprising an amino acid sequence selected from the sequences shown in SEQ ID NO: 1, SEQ ID NO: 2 and SEQ ID NO: 3, having no more than 8 amino acids.

2. A peptide according to claim 1, having an amino acid sequence selected from the sequences shown in SEQ ID NO: 4, SEQ ID NO: 5 and SEQ ID NO: 6.

3. A peptide according to claim 1, having an amino acid sequence selected from the sequences shown in SEQ ID NO: 1, SEQ ID NO: 2 and SEQ ID NO: 3.

4. A fragment of a peptide according to claim 3, having at least 3 amino acids.

5. A fragment of a peptide according to claim 4, having an amino acid sequence selected from the sequences shown in SEQ ID NO: 7, SEQ ID NO: 8 and SEQ ID NO: 9.

6. Acid or base addition salts, in particular pharmaceutically acceptable salts, esters, solvates and anhydrates of the peptides according to claims 1 to 5.

7. A composition comprising at least one peptide or derivative thereof defined in claims 1 to 6.

8. A pharmaceutical composition according to claim 7, **characterized in that** the derivatives defined in claim 6 are pharmaceutically acceptable, and **in that** a pharmaceutically acceptable carrier is present.

9. A pharmaceutical composition according to claim 7, **characterized in that** the peptides or derivatives thereof defined in claims 1 to 6 are present in an effective amount to reduce hypertension in mammals.

10. A food composition comprising at least one peptide or derivative thereof defined in claims 1 to 6.

11. A food composition according to claim 10, **characterized in that** the peptides or derivatives thereof defined in claims 1 to 6 are present in an effective amount to reduce hypertension in mammals.

12. A food composition according to claims 10 and 11, **characterized in that** said food composition is a food selected from: a beverage, infused food, milk, yogurt, cheese, flavored milk drink, bread, cake, butter, margarine, a sauce, a condiment, a salad dressing, fruit juice, syrup, a dessert, icings and fillings, a soft frozen product, a confection, a chewing gum and an intermediate food.

13. A protein hydrolysate, obtained from goat or sheep milk, enriched in at least one of the peptides or derivatives thereof defined in claims 1 to 6.

14. The protein hydrolysate of claim 13, wherein the combined amount of the peptides or salts thereof of claims 1 to 6 is between 0,1% and 5%, preferably between 1% and 2% (in dry weight) of the protein hydrolysate.

15. Use of a peptide according to claims 1 to 6 or of a protein hydrolysate according to claims 13 and 14 in the preparation of a dietary supplement, food ingredient or food composition.

16. A peptide or derivative thereof defined in claims 1 to 6 for use as a medicament.

17. A peptide or derivative thereof defined in claims 1 to 6 for use in the treatment or prophylaxis of hypertension, stroke, coronary disease, myocardial infarction, metabolic syndrome, peripheral vascular disease or abdominal aortic aneurysm in mammals.

18. A composition according to claims 7 to 12 and a hydrolysate according to claims 13 and 14 for use as a medicament.

19. A composition according to claim 7 to 12 and a hydrolysate according to claims 13 and 14 for use in the treatment or prophylaxis of hypertension, stroke, coronary disease, myocardial infarction, metabolic syndrome, peripheral vascular disease or abdominal aortic aneurysm in mammals.

20. Use of a peptide or derivative thereof defined in claims 1 to 6 in the manufacture of a medicament.

21. Use of a peptide or derivative thereof defined in claims 1 to 6 in the manufacture of a medicament for the treatment or prophylaxis of hypertension, stroke, coronary disease, myocardial infarction, metabolic syndrome, peripheral vascular disease or abdominal aortic aneurysm in mammals.

22. A process for the preparation of the peptides defined in claims 1, 2, 4 and 5 which comprises the steps of:
a) obtaining the casein fraction of goat or sheep milk;
b) hydrolyzing the casein fraction using a protease;
c) inactivating the protease;
d) isolating the peptides.

23. A process for the preparation of the peptides defined in claim 3 which comprises the steps of:
a) obtaining the casein fraction of goat or sheep milk;
b) hydrolyzing the casein fraction using a subtilisin;
c) inactivating the subtilisin;
d) isolating the peptides.

24. A process for the preparation of the protein hydrolysate defined in claims 13 and 14, comprising the steps of:
a) obtaining the casein fraction of goat or sheep milk;
b) hydrolyzing the casein fraction using a protease;
c) inactivating the protease;
d) enriching the mixture in the peptides defined in claims 1 to 6.

25. A process according to claim 24 which further comprises the step:
e) drying the hydrolysate to obtain a powder.

26. A process according to claims 24 and 25, **characterized in that** the protease is a subtilisin.

27. A process according to claim 26, **characterized in that** the subtilisin-comprises one or more compounds derived from a fermentation broth of a *Bacillus* species or one or more compounds derived from a cellular extract of the *Bacillus* species or a solid support immobilizing one or more compounds derived from a fermentation broth of the *Bacillus* species or a solid support immobilizing one or more compounds derived from a cellular extract of the *Bacillus* species.

28. The process according to claim 27, **characterized in that** the *Bacillus* species is *Bacillus amyloliquefaciens.*
